# NEW EUROPEAN PATENT SPECIFICATION

(11) **EP 1 171 471 B2**
(45) Date of publication and mention of the opposition decision: **08.07.2009**
(45) Mention of the grant of the patent: 17.12.2003
(21) Application number: 00918528.1
(22) Date of filing: 31.03.2000
(51) Int. Cl.: C08B 11/02, C08B 11/193, A23L 1/0534

(54) **ENHANCED GEL STRENGTH METHYLCELLULOSE**
METHYLCELLULOSE MIT VERBESSERTER GELFESTIGKEIT
METHYLCELLULOSE A FORCE DE GEL AMELIOREE

(30) Priority: 01.04.1999 US 283506; 01.04.1999 US 283921
(43) Date of publication of application: 16.01.2002
(73) Proprietor: DOW GLOBAL TECHNOLOGIES INC., Midland MI 48674 (US)
(72) Inventor: REIBERT, Kenneth, C., Baton Rouge, LA 70817 (US); CONKLIN, Jerry, R., Midland, MI 48642 (US)
(74) Representative: Raynor, John
(86) International application number: PCT/US2000/008660
(87) International publication number: WO 2000/059947

(56) References cited:
- DE-B- 1 060 374
- US-A- 2 160 782
- US-A- 5 618 800

## Description

The present invention relates to methylcelluloses having enhanced gel strength, processes for making such methylcelluloses, and compositions containing such methylcelluloses.

Cellulose ethers have been employed as additives to food compositions and processes to provide physical properties such as thickening, freeze/thaw stability, lubricity, moisture retention and release, film formation, texture, consistency, shape retention, emulsification, binding, suspension, and gelation. For example, methylcellulose has been utilized in food applications ever since it was discovered that the thermal gel property of certain methylcelluloses is similar to that of egg white. That is, solutions of methylcellulose undergo a heat-catalyzed gelation much like fresh raw egg white.

In fact, there has been a long felt need in the food industry for replacing egg whites in foods. This long felt need has been motivated by various reasons including health and religious reasons.

An important characteristic of both egg whites and methylcellulose is their ability to bind food ingredients together. It has been noted that the gel strength of these two food hydrocolloids is responsible for this binding force in foods.

Methylcellulose has partially met the industry's long felt need by replacing egg whites in certain food applications. However, the gel strength of methylcellulose has been insufficient to replace egg whites in other food applications. Accordingly, there has continued to be a long felt industry need to replace egg whites in certain food applications, which have heretofore not been replaceable by conventional (that is, known in the art) methylcelluloses.

It is generally known in the art that methylcelluloses having higher viscosity also have higher gel strength. The present invention is directed to methylcelluloses that exhibit significantly greater gel strength at a given viscosity than any methylcellulose of the prior art. Methylcelluloses of the present invention enable the development of food compositions with superior binding, consistency, and shape retention.

The present invention allows one of ordinary skill in the art to use a methylcellulose with greater gel strength at a given viscosity. The present invention also allows one of ordinary skill in the art to use a methylcellulose with a lower viscosity at a given gel strength. Methylcelluloses having higher molecular weight (that is, viscosity) tend to bind moisture too tightly, which can give rise to a final formed food product having a dry feel to the mouth. The present invention allows one of ordinary skill in the art to improve moisture release and overall texture of a formed food product by using a methylcellulose with lower viscosity while maintaining the required gel strength.

In a first embodiment, the present invention is directed to a methylcellulose having a gel strength ("G'") greater than 223 x (v^{0.273}), where v represents the viscosity of the methylcellulose for a 2 percent aqueous solution at 20°C. This relationship of gel strength to viscosity can be represented by the equation G' ≥ 223 × (v^{0.273}), where "≥" means "greater than or equal to. Methylcelluloses of the present invention also have a methoxyl substitution of 21 percent to 42 percent based upon the weight of the methylcellulose.

Gel strengths can be measured by dynamic rheometry. The elastic modulus component of the complex viscosity is quantified in measuring gel strength. This dynamic elastic modulus represents the force component known generally as gel strength (G'). Techniques for measuring elastic modulus (storage modulus) are described in Kinetics of Thermal Gelation of Methylcellulose and Hydroxypropylmethylcellulose in Aqueous Solutions, Carbohydrate Polymers, volume 26, no. 3, pp. 195-203, which is incorporated herein by reference. Unless stated otherwise, all gel strengths in the present specification were determined by measuring the elastic modulus of a 1.5 weight percent aqueous solution of the methylcellulose in a dynamic rheometer.

In a second embodiment, the present invention is directed to a process for making methylcelluloses having a level of methoxyl substitution of 21 to 42 weight percent. The process comprises the steps of: a) reacting a cellulose pulp with a first amount of aqueous alkali and a first amount of a methylating agent at reaction conditions sufficient to methylate the cellulose pulp to a first level of methoxyl substitution which is 20 percent or more of the total level of methoxyl substitution; and b) reacting the methylcellulose of first level of methoxyl substitution with a second amount of aqueous alkali and adding to the resulting methyl cellulose continuously or incrementally a second amount of a methylating agent at reaction conditions sufficient to methylate it to a second level of methoxyl substitution which is about 40 percent or more of the total level of methoxyl substitution, such that the process is a two-stage process, 20 to 80 percent of the total level of methoxyl substitution is carried out in the first stage and 80 to 20 percent of the total level of methoxyl substitution is carried out in the second stage.

A third embodiment of the present invention is directed to a process for making methylcellulose having a level of methoxyl substitution of 21 to 42 weight percent comprising the steps of: a) reacting a cellulose pulp with a first amount of aqueous alkaline hydroxide and a first amount of a methylating agent at reaction conditions sufficient to methylate the cellulose pulp to a first level of methoxyl substitution which is 20 percent or more of the total level of methoxyl substitution; and b) reacting the methylcellulose of first level of methoxyl substitution with a second amount of aqueous alkaline hydroxide to alkalize it to a second level of alkalization and adding to the methyl cellulose of second level of alkalization continuously or incrementally a second amount of a methylating agent at reaction conditions sufficient to methylate it to a second level of methoxyl substitution which is 40 percent or more of the total level of methoxyl substitution, and the process is a three-stage process, the process comprising the additional steps of:
contacting the methyl cellulose of the second level of methoxyl substitution with a third amount of aqueous alkaline hydroxide to alkalize it to a third level of alkalization; and
contacting the methylcellulose of the third level of alkalization with a third amount of methylating agent.

A fourth embodiment of the present invention is directed to food compositions comprising a methylcellulose of the present invention.

A fifth embodiment of the present invention is directed to a pharmaceutical capsule comprising a methylcellulose of the present invention.

The Figure is a graphical representation depicting the elastic modulus of methylcelluloses of the present invention and those of the prior art.

The present invention provides novel methylcelluloses having elevated gel strength for a given viscosity grade and percent methoxyl substitution compared to that of conventional methylcelluloses. Preferred methylcelluloses of the present invention may also exhibit lower gelation temperatures than conventional methylcelluloses of equivalent viscosity and percent methoxyl substitution. Preferred methylcelluloses of the present invention may also exhibit longer meltback times than conventional methylcelluloses of equivalent viscosity and substitution.

Lower gelation temperature may be desired or preferred in some applications, but is a non-essential property of the present invention. Reduced gelation temperature is useful in food manufacturing and processing. Food compositions can be gelled at lower temperatures saving energy and processing time during heating/cooling cycles. Further, food compositions can retain form at broader temperature ranges during processing. Gelation temperature is determined by heating a 1.5 percent by weight aqueous solution of the methylcellulose and observing the narrow temperature range at which gelation takes place.

Meltback time generally refers to the length of time required for a thermally formed gel of a methylcellulose to melt while cooling to an ambient temperature. Longer meltback time may be desired or preferred for some applications, but are a non-essential property of the present invention. Longer meltback time at ambient temperature is useful in food processing and manufacturing. Gelation can be maintained over a wider temperature range and longer and better retention of texture during food processing and consumption are possible. Meltback time is determined according to the following: provide 15 grams of a 1.5 weight percent of an aqueous solution of the methylcellulose in a 20 milliliter beaker, heat the solution for 8 minutes in boiling water - the solution will gel in the beaker; invert the beaker onto a flat surface in an ambient temperature environment; allow the gel to cool and subsequently melt to form a puddle on the surface. Meltback time is measured from the time cooling begins (removal from the boiling water) to when a clear puddle forms.

The methylcelluloses of the present invention are useful in the same applications wherein conventional methylcelluloses are currently utilized, as well as additional applications where conventional methylcelluloses are not currently effective. Methylcelluloses of the present invention are particularly useful in food compositions and pharmaceutical capsules.

Methylcelluloses having viscosities of up to about 1,000,000 centipoise (cP) in a two percent aqueous solution at 20°C can be prepared according to the present invention. Preferred methylcelluloses may have viscosities of about 1 to about 600,000 cP (two percent solution 20°C). More preferred methylcelluloses may have viscosities of about 1 to about 100,000 cP (two percent solution at 20°C). For purposes of the present specification, all viscosities of aqueous solutions are determined by Ubbelohde tube according to ASTM D1347-72 and D2363-79 (unless stated otherwise).

Methylcelluloses that can be produced according to the present invention include, but are not limited to, hydroxyethylmethylcellulose ("HEMC"), hydroxypropylmethylcellulose ("HPMC"), hydroxybutylmethylcellulose ("HBMC"), methylethylcellulose ("MEC"), and carboxymethylmethylcellulose ("CMMC"). Preferred HPMCs of the present invention will have a hydroxypropyl substitution of from about 1 to about 32 percent, more preferably from about 1 to about 14 percent, and most preferably from about 3 to about 12 percent. Methylcelluloses preferred for use in food compositions will have a non-methoxyl substitution content or level of about 5 percent or less, more preferably about 1 percent or less, and even more preferably about 0.2 percent or less. Methylcelluloses most preferred for use in food compositions will be substantially free of non-methoxyl substitution content. For purposes of the present specification, all non-methoxyl substitutions are expressed in weight percent based upon the weight of the methylcellulose (unless stated otherwise).

Methylcelluloses of the present invention that are useful in food compositions have a methoxyl substitution of 21 to 42 weight percent based upon the weight of the methylcellulose. For purposes of the present specification, all methoxyl substitutions are expressed in weight percent based upon the weight of the methylcellulose (unless stated otherwise). For purposes of the present specification, all methoxyl substitution is determined according to ASTM D2363-72 (unless stated otherwise).

Preferred (for purposes of use in food compositions) methylcelluloses have a methoxyl substitution of at least about 25 percent. More preferred methylcelluloses have a methoxyl substitution of at least about 29 percent. Preferred (for purposes of use in food compositions) methylcelluloses of the present invention have a methoxyl substitution of less than about 35 percent. More preferred methylcelluloses have a methoxyl substitution of less than about 32 percent.

Methylcelluloses of the present invention are produced by a novel process. It is well known in the art to alkylate cellulose to produce ethers (such as methylcellulose). In conventional processes, cellulose pulp is completely alkalized with sodium hydroxide and etherified (that is, methylated) with a methylating agent (usually methyl chloride) in a single stage. The degree to which the cellulose is methylated is referred to as the percent methoxyl substitution. This process and the underlying chemistry has been well known in the art for a long time.

Contrary to conventional processes for producing methylcellulose, the methylcelluloses of the present invention are produced via a novel process that methylates cellulose pulp in two or more separate stages. According to the present invention, cellulose pulp is partially alkalized with alkaline hydroxide and partially methylated with a methylating agent (preferably methyl chloride) in a first stage. While the present invention should not be limited by any particular theory, it is believed that the methylated sites on the cellulose after the first stage are more selective for methylating in subsequent stages. That is, methylation in subsequent stages is more likely to occur near the sites that were methylated in the first stage, causing a less uniform distribution of methylated sites on the cellulose than occurs in conventional methylcelluloses. This less uniform distribution can be referred to as "blocky" substitution. The partially methylated cellulose produced in the first stage is further alkalized and further etherified with additional alkaline hydroxide and methyl chloride to the desired level of completion in a later stage or stages. The methylating agent utilized in subsequent stages is introduce via continuous addition or incremental addition at reaction conditions sufficient to produce the desired level of methoxyl substitution.

The cellulose utilized in producing methylcelluloses of the present invention is typically cellulose pulp obtained from wood or cotton. The pulp is preferably provided in a powder or chip form. Wood pulp is preferred.

The alkylating of the cellulose pulp is carried out in a stepwise manner in stages. A "stage" refers to a reaction sequence in which an alkalization reaction and a methylation reaction (or substitution) takes place. A stage effectively increases the level of methoxyl substitution of the cellulose pulp or partially methylated cellulose. Optionally, other types of etherification such as hydroxypropyl substitution can be effected along with or in addition to methoxyl substitution.

According to the present invention, cellulose pulp is alkalized in two or more stages in one or more reactors with an aqueous alkali, preferably aqueous sodium hydroxide. The pulp may be alkalized with alkaline hydroxide by any means known in the art such as steeping in a bath or stirred tank containing aqueous alkali or spraying aqueous alkali directly on dry pulp. Reaction time varies according to hydroxide concentration, temperature, and retention time. The aqueous alkali is preferably used at an alkaline hydroxide content of about 30 to about 70 percent by weight based upon the weight of the water. The temperature of alkalization preferably ranges from about 30°C to about 110°C and most preferably about 30°C to about 90°C. Uniform swelling and alkali distribution in the pulp may be controlled by mixing and agitation. The rate of addition of aqueous alkali may be governed by the ability to cool the reactor during the exothermic alkalization reaction. The rate of addition of aqueous alkali is not critical to the present invention. If desired, an organic solvent such as dimethyl ether may be added to the reactor as a diluent and a coolant. If desired, the headspace of the reactor or reactors may be evacuated or purged with an inert gas such as nitrogen to control oxygen-catalyzed depolymerization of the alkali cellulose.

According to the present invention, alkalized cellulose pulp is etherified (that is, methylated) in two or more stages in one or more reactors to form methylcellulose. Reaction time for etherification will depend on concentration, pressure, temperature, retention time, and the ability to control the exothermic reaction. The preferred etherifying agent is a methylating agent such as methyl chloride or dimethyl sulfate. Methyl chloride is preferred. The methylating agent may be added via batch load addition, continuous addition, or incremental addition in one or more of the stages. The methylating agent is added via continuous addition or incremental addition in at least one stage after the first stage, preferably in the second stage. "Batch load addition" means addition substantially without pause over a relatively short period of time. "Continuous addition" means addition substantially without pause over a longer period of time. "Incremental addition" means periodic addition of smaller, discrete amounts over a longer period of time. The alkaline hydroxide and the methylating agent may be added to the reactor at the same time but are preferably added sequentially with the alkaline hydroxide being added first and the methylating agent second.

A two-stage process is used in one embodiment for making methylcelluloses of the present invention. In stage one, the aqueous alkaline hydroxide and the methylating agent are reacted with cellulose pulp to form a methylcellulose of a first level of methoxyl substitution. Each of the alkaline hydroxide and the methylating agent may be added in stage one via a batch load addition, continuous addition, or incremental addition. The rate of addition is not critical. The reaction temperature in the first stage is preferably controlled so that generally uniform contact and reaction can occur between the alkaline hydroxide, the methylating agent, and the cellulose pulp. In the second stage, additional amounts of the aqueous alkaline hydroxide and the methylating agent are reacted with the partially methylated cellulose to form a methylcellulose with a second level (that is, desired level) of methoxyl substitution. The alkaline hydroxide and the methylating agent may be added in the second stage via a batch load addition, continuous addition, or incremental addition. The rate of addition of hydroxide in the second stage is not critical.

The methylating agent is added in the second stage at a reaction mass temperature of about 65°C to about 120°C over an elapsed addition of time of 15 minutes or more; preferably at about 75°C to about 100°C at 20 minutes or more; and most preferably at about 80°C to about 90°C at 25 minutes or more. Although the methylating agent can be added continuously or incrementally over any extended period of time in the second stage, it is preferred for reasons of time economy to carry out the addition in about 120 minutes or less, more preferably in about 60 minutes or less, and most preferably in about 25 to about 45 minutes. After addition of the methylating agent in the second stage, etherification can be carried out at any temperature at which the reaction can proceed, but it is preferred for reason of time economy to carry it out at about 65°C to about 120°C and more preferably from about 80°C to about 90°C. Temperature within the reactor can be determined by any means known in the art. Temperature determination means include using vapor temperature control and using a thermocouple which protrudes into the contents (cellulose pulp/methylcellulose mass) of the reactor. In a preferred two-stage process, both stages are carried out in the same reactor.

Preferably, about 20 to about 80 percent of the total methoxyl substitution is carried out in the first stage and about 80 to about 20 percent in the second stage. More preferably, about 30 to about 70 percent of the total methoxyl substitution is carried out in the first stage and about 70 to about 30 percent in the second stage. Most preferably, about 40 to about 60 percent of the total methoxyl substitution is carried out in the first stage and about 60 to about 40 percent in the second stage. Some embodiments of two-stage processes are described in Table 1.

A three-stage process is used in another embodiment for making the present methylcellulose. The first stage is carried out in a manner similar to that of the first stage of the two-stage process described above. Either or both of the second and third stages are carried out in the same manner as the second stage in the two-stage process described above (the methylating agent is added continuously or incrementally over a period of time). In a preferred three-stage process, about 20 to about 60 percent of the total methoxyl substitution is carried out in each of the first and second stages and about 5 to about 30 percent in the third stage. Some embodiments of three-stage processes are described in Table 1.

It is also possible to have processes with four or more stages. The first stage of such a process would be carried out in the same manner as the first stage in the two-stage process described above. One or more of the subsequent stages would be carried out in the same manner as the second stage in the two-stage process described above (the methylating agent is added continuously or incrementally over a period of time).

**Table 1**

| Some Useful Embodiments of the Process of the Present Invention | | | | | | | | |
|---|---|---|---|---|---|---|---|---|
| | Embodiments | | | | | | | |
| Features | #1 | #2 | #3 | #4 | #5 | #6 | #7 | #8 |
| partial alkalization in first stage; addition of alkaline hydroxide | X | X | X | X | X | X | X | X |
| partial etherification in first stage; batch load addition of methylating agent | X | X | | | X | X | | |
| partial etherification in first stage; continuous/incremental addition of methylating agent | | | X | X | | | X | X |
| partial alkalization in second stage; addition of alkaline hydroxide | X | X | X | X | X | X | | |
| partial etherification in second stage; continuous/incremental addition of methylating agent | X | X | X | X | X | X | | |
| partial etherification in second stage; batch load or continuous/incremental addition of methylating agent | | | | | | | X | X |
| partial alkalization in third stage; addition of alkaline hydroxide | | | | | X | X | X | X |
| partial etherification in third stage; continuous/incremental addition of methylating agent | | | | | | | X | X |
| partial etherification in third stage; batch load or continuous/incremental addition of methylating agent | | | | | X | X | | |
| single reactor for all stages | X | | X | | X | | X | |
| separate reactor for each stage | | X | | X | | X | | X |

Methylcelluloses such as HEMC, HPMC, HBMC, MEC, and CMMC can be prepared by reacting the cellulose pulp or a methylcellulose with another etherifying agent in addition to the methylating agent (also an etherifying agent). Useful etherifying agents include ethyl chloride, ethylene oxide, propylene oxide, and butylene oxide. The other etherifying agent can be reacted in any stage before, during, or after reaction by the methylating agent at process conditions sufficient to effect the desired reaction. The other etherifying agent may be added to the reactor via a batch load addition, continuous addition, or incremental addition. Preferably, the other etherifying agent is reacted in the first stage. Preferably, the other etherifying agent is reacted before or along with the methylating agent.

The methylating agent and any other etherifying agent may be added to a reactor in a liquid or vapor form. Liquid form is highly preferred. The reactor is preferably maintained at pressures such that the agents remain predominantly in liquid phase.

After etherification, the methylcellulose is washed to remove salt and other reaction by-products. Any solvent in which salt is soluble may be employed, but water is preferred. The methylcellulose may be washed in the reactor, but is preferably washed in a separate washer located downstream of the reactor. Before or after washing, the methylcellulose may be stripped by exposure to steam to reduce residual organic content.

The methylcellulose is dried to a reduced moisture and volatile content of preferably about 0.5 to about 10.0 weight percent water and more preferably about 0.8 to about 5.0 weight percent water and volatiles based upon the weight of methylcellulose. The reduced moisture and volatiles content enables the methylcellulose to be milled into particulate form. The methylcellulose is preferably dried at a temperature of from about 40°C to about 130°C. Useful dryers include tray dryers, fluid bed dryers, flash dryers, agitation dryers, and tube dryers.

The methylcellulose is milled to particulates of desired size. If desired, drying and milling may be carried out simultaneously. Milling may be accomplished by any means known in the art such as a ball mill, an impact pulverizer, knife grinder, and air-swept impact mill.

The present methylcellulose is useful in a variety of food compositions. Examples of food compositions include vegetable, meat, and soy patties; reformed seafood; reformed cheese sticks; cream soups; gravies and sauces; salad dressing; mayonnaise; onion rings; jams, jellies, and syrups; pie filling; potato products such as french fries and extruded fries; batters for fried foods, pancakes/waffles, and cakes; pet foods; beverages; frozen desserts; cultured dairy products such as ice cream, cottage cheese, yogurt, cheeses, and sour creams; cake icing and glazes; whipped topping; leavened and unleavened baked goods; and the like. In forming food compositions, the methylcellulose is typically admixed with foodstuffs during the process and formation of the compositions. The foodstuffs may be in any known form such as particle form or unitary form. Excellent teachings to the preparation of food compositions with methylcellulose are found in the following: METHOCEL® (trademark of The Dow Chemical Company) product publications: METHOCEL Premium Food Gums, Form Nos. 192-1037-87, 192-1047-87, 192-1046-87, 192-1051-87, 192-1050-87, 192-1049-87, 192-1053-87, 192-982-87, 192-979-87, 192-985-87, 192-1054-87, 192-1048-87, 192-987-87, 192-986-87, 192-989-87, 192-988-87, 192-984-87, 192-983-87, 192-981-87, 192-991-87, 192-980-87, 192-990-87, and 192-1052-87 (all published in 1987); Selecting METHOCEL Food Gums, Form No. 192-855-1281R (published in 1981); METHOCEL Food Gums In Non-Dairy Whipped Topping, Form No. 192-877-482 (published in 1982); METHOCEL Food Gums In Fried Foods, Form Nos. 192-875-482 and 192-881-482 (all published in 1982); METHOCEL Food Gums In Salad Dressings and Sauces, Form Nos. 192-876-482, 192-880-482, and 192-905-1282 (all published in 1982); and METHOCEL Food Gums In Bakery Products, Form Nos. 192-874-482 and 192-878-482 (all published in 1982).

Methylcelluloses particularly useful in food compositions are methylcelluloses having little or no non-methoxyl substitution and hydroxypropylmethylcellulose. Methylcelluloses are typically used in food compositions at levels of about 0.01 to about 5 percent based upon the total weight of the food composition.

Methylcelluloses are useful in other applications such as building products, industrial products, agricultural products, personal care products, household products, and pharmaceutical products. Useful pharmaceutical applications include as capsules, encapsulants, tablet coatings, and as an excipient for medicaments and drugs. Useful excipient functions include as sustained-release and timed-release tablets. Useful building applications include drywall tape-joint compounds, mortars, grouts, cement plasters, spray plasters, cement stucco, adhesives, pastes, and wall/ceiling texturizers. Useful industrial applications include binders and processing aids for tape casting, extrusion forming, and injection molding and ceramics. Useful agricultural applications include spray adherents and suspending/dispersing aids for pesticide, herbicide, and fertilizer powders. Useful personal care and household products include shampoos, lotions, creams, and cleaning products.

The present methylcelluloses are particularly useful in compositions for pharmaceutical capsules. Capsules formed from the present methylcelluloses may exhibit substantially less distortion after drying than capsules formed from conventional methylcelluloses. Particularly useful methylcelluloses are methylcellulose having little or no non-methoxyl substitution and hydroxypropylmethylcellulose of low molecular weight, i.e. about 3 to about 100 cP and preferably about 3 to about 15 cP in a two percent aqueous solution. Low molecular weight methylcelluloses can be prepared directly from the processes described above or can be prepared from high molecular weight methylcelluloses via acid-catalyzed depolymerization. Useful acids include anhydrous hydrogen chloride and hydrochloric acid. Following depolymerization to the desired degree, the acid is neutralized and the depolymerization stopped by contact with a base such as sodium bicarbonate. Useful teachings relating to making low molecular weight methylcelluloses are seen in U.S. Serial No. 09/203,324.

Methylcellulose capsules are typically manufactured by dipping hot pins in a cold, aqueous methylcellulose coating solution or by dipping cold pins in a hot, aqueous methylcellulose coating solution. The solutions gel on the pins and water evaporates during a drying step to form thin film layers of dried methytcellulose around the pins. The thin films take the form of caps and bodies, which are removed from the pins and mated to form capsules. Processes for making capsules are seen in U.S. Patent Nos. 3,617,588; 4,001,211; 4,917,885; and 5,756,036.

When drying takes place non-uniformly during the manufacture of pharmaceutical capsules with conventional methylcelluloses, caps and bodies can sometimes become distorted and difficult to mate or assemble into capsules. Caps and bodies formed from the present methylcelluloses may better resist such distortion because of their enhanced gel strength.

The following are examples of the present invention. Unless otherwise indicated, all percentages, parts, and proportions are by weight.

### EXAMPLES

### Example 1

A methylcellulose of the present invention was made in accordance with a process of the present invention.

Finely ground cellulose wood pulp was loaded into a jacketed, agitated reactor. The reactor was evacuated and purged with nitrogen to remove oxygen and then evacuated again. The reactor was used in two stages. In the first stage, 50 percent sodium hydroxide in water by weight was sprayed onto the cellulose at a weight ratio of 0.45/1.0 NaOH/cellulose and the temperature adjusted to 40°C (starting temperature). After stirring the NaOH/cetlulose for about 10-20 minutes, a mixture of dimethyl ether and methyl chloride was added to the reactor with additional methyl chloride so that the weight ratio of methyl chloride/cellulose was about 0.64/1.0. The contents of the reactor were then heated from 40°C to 80°C over the next 40 minutes. After reaching 80°C (cook temperature), the first stage reaction was allowed to proceed for another 30 minutes (cook time). The second stage was effected by adding the remainder of the sodium hydroxide and methyl chloride and allowing for additional reaction. A second quantity of 50 percent NaOH in water by weight was added over 10 minutes at a weight ratio of 0.65/1.0 NaOH/cellulose (the cellulose is actually partially etherified at this point in time). A second quantity of methyl chloride was added over about 35 minutes to a level of 0.90/1.0 weight ratio of methyl chloride/cellulose. The reaction was continued at 80°C for an additional 30 minutes (cook time) to complete the etherification. Table 2 depicts process information and data pertaining to alkalization and etherification.

After the reaction, the reactor was vented and cooled to 50°C. The contents of the reactor were removed and transferred to a tank containing hot water to form a slurry, which was subsequently agitated for 15 minutes. This slurry was pumped from the hot tank to a filter where it was de-watered and washed with hot water to remove the salt and organic by-products. The wet methylcellulose was then transferred to a dryer where moisture and volatiles content was reduced to 1 to 4 weight percent based upon the weight of the methylcellulose. The methylcellulose was then ground to a particle size of about 40 mesh (420 micrometers).

The methylcellulose product was analyzed and found to contain 31.8 percent methoxyl substitution (a methoxyl degree of substitution of 1.96). It exhibited a viscosity of 17,000 centipoise (cP) in a 2 percent by weight aqueous solution by weight, a gelation temperature (T_{gel}) of 105°F - 108°F (40.6°C - 42.2°C), and a elastic modulus (G') of 5445 Pascals for a 1.5 percent by weight aqueous solution, and a meltback time of 35 minutes. Product properties are set forth in Table 3. These physical properties are more desirable than those of conventional methylcelluloses of similar substitution and viscosity level. Such conventional methylcelluloses typically exhibit a T_{gel} of about 52° - 59°C for a 1.5 percent aqueous solution and a G' of 800-2000 Pascals for a 1.5 percent aqueous solution. Thus, the methylcellulose of the present invention has the advantages of both a significantly higher G' and a significantly lower gelation temperature compared to a conventional methylcellulose of similar substitution and viscosity grade.

In the various examples disclosed herein, G' was measured for a 1.5 percent by weight aqueous solution in a Bohlin VOR Rheometer (Bohlin Corp.) with C25 serrated bob and cup system.

### Examples 1A-1D

Samples of the methylcellulose product of Example 1 were depolymerized by reaction with anhydrous hydrogen chloride for varying lengths of time followed by neutralization with sodium bicarbonate. After depolymerization, a sample exhibited a viscosity of 614 cP (two percent solution), a G' of 2250 Pascals and a meltback time of 20 minutes. After another depolymerization, a sample exhibited a viscosity of 219 cP, a G' of 1400, a T_{gel} of 108°F (42°C), and a meltback time of 40 minutes. After another depolymerization, a sample exhibited a viscosity of 81 cP, a G' of 1390 Pascals, a T_{gel} of 103°F (39°C) and a meltback time of 19 minutes. After another depolymerization, a sample exhibited a viscosity of 66 cP, a G' of 1680 Pascals, and a meltback time of 25 minutes. Product properties are set forth in Table 4.

### Example 2

Another methylcellulose of the present invention was made in accordance with a process of the present invention. The process is as in Example 1 except where indicated otherwise in Table 2.

The methylcellulose product had a methoxyl substitution of 31.3 percent and exhibited a viscosity of 26,000 cP in a 2 percent aqueous solution by weight, a T_{gel} of 34.4°C, a G' of 3740 Pascals, and a meltback time of greater than 50. These physical properties compare very favorably with those of a conventional methylcellulose of similar substitution and viscosity level. Product properties are set forth in Table 3.

### Examples 2A-2B

Samples of the methylcellulose end product of Example 2 were depolymerized by reaction with anhydrous hydrogen chloride for varying lengths of time followed by neutralization with sodium bicarbonate. After depolymerization, a sample exhibited a viscosity of 357 cP (2 percent solution), a G' of 1080 Pascals, and a meltback time greater than 50 minutes. After another depolymerization, a sample exhibited a viscosity of 29 cP, a G' of 569 Pascals, a T_{gel} of 87°F (31 °C), and a meltback time of 30 minutes. Product properties are set forth in Table 4.

### Example 3

Another methylcellulose of the present invention was made with the process of the present invention. The process is as in Example 1 except where indicated otherwise in Table 2.

The methylcellulose product had a 29.9 percent methoxyl substitution and exhibited a viscosity of 30,000 cP (2 percent solution), a T_{gel} of 89°F (31.7°C), and a G' of 3200 Pascals, and a meltback time greater than 50 minutes. Product properties are set forth in Table 3.

### Example 4

Another methylcellulose of the present invention was made with the process of the present invention. The process is as in Example 1 except where indicated otherwise in Table 2.

The methylcellulose product had a 32.9 percent methoxyl substitution and exhibited a viscosity of 11,000 cP (2 percent solution), a T_{gel} of 122°F (50°C), a G' of 3180 Pascals, and a meltback time of 17 minutes. Product properties are set forth in Table 3.

### Example 5

Another methylcellulose of the present invention was made with the process of the present invention. The process is as in Example 1 except where indicated otherwise in Table 2.

The methylcellulose product had a 32.7 percent methoxyl substitution and exhibited a viscosity of 2600 cP (2 percent solution), a T_{gel} of 118°F (48°C), a G' of 1460 Pascals and a meltback time of 20 minutes. Product properties are set forth in Table 3.

### Example 6

Another methylcellulose of the present invention was made with the process of the present invention. The process is as in Example 1 except where indicated otherwise in Table 2.

The methylcellulose product had a 26.9 percent methoxyl substitution and exhibited a viscosity of 330 cP in a 2 percent aqueous solution by weight, a T_{gel} of 128°F (54°C), a G' of 1470 Pascals and a meltback time of 8 minutes. Product properties are set forth in Table 3.

### Example 7

Another methylcellulose of the present invention was made with the process of the present invention. The process is as in Example 1 except it has an additional (third) stage. Process information is set forth in Table 5.

The methylcellulose product had a 35.4 percent methoxyl substitution and exhibited a viscosity of 461,000 cP (2 percent solution), a T_{gel} of 45°C, and a G' of 6900 Pascals and was stable to meltback at ambient temperature. This methylcellulose product has the advantages of a significantly higher G' and a significantly lower T_{gel} compared to conventional methylcelluloses of similar methoxyl substitution and viscosity level. Product properties and composition are set forth in Table 6.

### Example 8

Another methylcellulose of the present invention was made with the process of the present invention. The process is as in Example 1 except it has an additional (third) stage. Process information is set forth in Table 5.

The methylcellulose product had a 36.1 percent methoxyl substitution and exhibited a viscosity of 26,000 cP (2 percent solution), a T_{gel} of 113°F (45°C), a G' of 7990 Pascals, and was stable to meltback at ambient temperature. This methylcellulose product has the advantages of a significantly higher G' and a significantly lower gelation temperature compared to conventional methylcelluloses of similar methoxyl substitution and viscosity level. Product properties and composition are set forth in Table 6.

### Example 9

Another methylcellulose of the present invention was made with the process of the present invention. The process is as in Example 1 except it has an additional (third) stage. Process information is set forth in Table 5.

The methylcellulose product had a 34.9 percent methoxyl substitution and exhibited a viscosity of 25,000 cP (2 percent solution), a T_{gel} of 95°F(35°C), and a G' of 7565 Pascals and was stable to meltback at ambient temperature. This methylcellulose product has the advantages of a significantly higher G' and a significantly lower T_{gel} compared to conventional methylcelluloses of similar methoxyl substitution and viscosity level. Product properties and composition are set forth in Table 6.

A number of methylcellulose samples were generated using a conventional process known in the art. The product properties of these samples are set forth in Table 7.

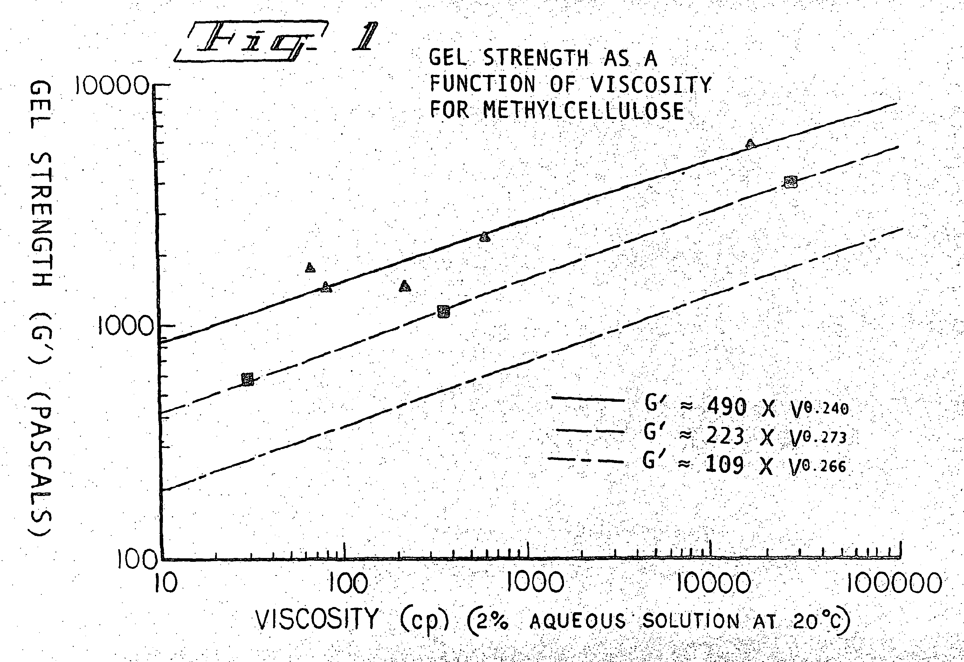

The gel strength and viscosity properties of the prior art samples set forth in Table 7 were graphed along with the gel strength and viscosity properties of examples 1, 1A, 1B, 1C, 1D, and examples 2, 2A, and 2B as set forth in Tables 3 and 4. The corresponding graph is shown in Figure 1. The relationship between gel strength (expressed on logarithmic scale) and viscosity (logarithmic scale) is approximately linear. Lines were drawn for each set of data using a best fit algorithm. The equations approximating each line are indicated on the graph.

The graph in Figure 1 demonstrates that the gel strength of methylcelluloses of the present invention having methoxyl substitution of between about 29 percent and 32 percent is greater than approximately 223 x v^{0.273}. It should be understood that this gel strength to viscosity relationship is an approximation and methylcelluloses of the present invention may fall slightly below this approximation. For example, the data from Table 3 indicates that Example 3 falls slightly below the line representing Examples 2, 2A, and 2B, but is well above the line representing the prior art.

**Table 2**

| Process Information for Examples 1-6 | | | | | | |
|---|---|---|---|---|---|---|
| | Example 1 | Example 2 | Example 3 | Example 4 | Example 5 | Example 6 |
| NaOH/Cellulose Ratio (First Stage) | 0.45 | 0.45 | 0.50 | 0.45 | 0.44 | 0.42 |
| Starting Temperature (First Stage) | 40°C | 40°C | 40°C | 40°C | 40°C | 40°C |
| MeCl/cellulose Ratio (First stage) | 0.64/1.0 | 0.64/1.0 | 0.74/1.0 | 0.64 | 0.62 | 0.60 |
| Cook Temperature (First Stage) | 80°C | 80°C | 80°C | 80°C | 80°C | 80°C |
| Cook Time After Reaching Cook Temperature (Minutes) (First Stage) | 30 | 30 | 20 | 30 | 30 | 30 |
| Starting Temperature (Second Stage) | 80°C | 80°C | 80°C | 80°C | 80°C | 80°C |
| NaOH/Cellulose Ratio (Second Stage) | 0.65 | 0.65 | 0.40 | 0.65 | 0.65 | 0.60 |
| MeCl/Cellulose Ratio (Second Stage) | 0.90/1.0 | 0.90/1.0 | 0.61 | 0.90 | 0.90 | 0.82 |
| MeCl Feed Rate (Minutes) (Second Stage) | 30-35 | 30-35 | 30-35 | 30-35 | 30-35 | 30-35 |
| Cook Temperature (Second Stage) | 80°C | 80°C | 80°C | 80°C | 80°C | 80°C |
| Cook Time (Minutes) (Second Stage) | 60 | 60 | 60 | 60 | 60 | 60 |

**Table 3**

| Product Properties and Composition of Examples 1-6 | | | | | | |
|---|---|---|---|---|---|---|
| | Example 1 | Example 2 | Example 3 | Example 4 | Example 5 | Example 6 |
| Meo % | 31.8 | 31.3 | 29.9 | 32.9 | 32.7 | 26.9 |
| MeO DS | 1.96 | 1.91 | 1.81 | 2.03 | 2.01 | 1.61 |
| Viscosity (cP) | 17,000 | 26.000 | 30,000 | 11,000 | 2600 | 330 |
| T_{gel} | 105°-108°F (40.6°C - 42.2°C) | 94°F (34.4°C) | 89°F (31.7°C) | 122°F (50°C) | 118°F (48°C) | 128°F (54°C) |
| G' (Pascals) | 5445 | 3423 | 3200 | 3180 | 1460 | 1470 |
| Meltback time (minutes) | 35 | >50 | >50 | 17 | 20 | 8 |

| | | | | | | |
|---|---|---|---|---|---|---|
| G' - elastic modulus in Pascals in a 1.5 percent aqueous solution; corresponds to gel strength. Viscosity - viscosity in 2 percent aqueous solution in centipoise (cP). MeO % - percent methoxyl substitution based upon weight of methylcellulose. MeO DS - methoxyl degree of substitution. T_{gel} - gelation temperature | | | | | | |

**Table 4**

| Product Properties of Examples 1A-1D and Examples 2A-2B | | | | | | |
|---|---|---|---|---|---|---|
| | Ex. 1A | Ex. 1B | Ex. 1C | Ex. 1D | Ex. 2A | Ex. 2B |
| Viscosity (cP) | 614 | 219 | 81 | 66 | 357 | 29 |
| T_{gel} | 111°F(44°C) | 108°F (42°C) | 103°F(39°C) | 102°F(39°C) | 96°F(36°C) | 87°F (31°C) |
| G' (Pascals) | 2250 | 1400 | 1390 | 1680 | 1080 | 569 |
| Meltback Time (Minutes) | 20 | 40 | 19 | 25 | 50 | 30 |

**Table 5**

| Process Information for Examples 7-9 | | | |
|---|---|---|---|
| | Example 7 | Example 8 | Example 9 |
| NaOH/Cellulose Ratio (First Stage) | 0.55 | 0.55 | 0.55 |
| Starting Temperature (First Stage) | 30°C | 30°C | 30°C |
| MeCl/Cellulose Ratio (First Stage) | 0.71/1.0 | 0.70/1.0 | 0.70/1.0 |
| Cook Temperature (First Stage) | 80°C | 75°C | 75°C |
| Cook Time (Minutes) (First Stage) | 60 | 60 | 60 |
| NaOH/Cellulose Ratio (Second Stage) | 1.0 | 1.0 | 1.0 |
| MeCl/Cellulose Ratio (Second Stage) | 1.3/1.0 | 1.3/1.0 | 1.30 |
| MeCl Feed Rate (Minutes) (Second Stage) | 30-35 | 30-35 | 30-35 |
| Cook Temperature (Second Stage) | 80°C | 80°C | 80°C |
| Cook Time (Minutes) (Second Stage) | 90 | 90 | 90 |
| Venting of Reactor Headspace After Completion of Second Stage | Yes | Yes | Yes |
| NaOH/Cellulose Ratio (Third Stage) | 1.0/1.0 0 | 1.0/1.0 | 1.0/1.0 |
| Starting Temperature (Third Stage) | 60°C | 60°C | 80°C |
| MeCl/Cellulose Ratio (Third Stage) | 1.4/1.0 | 1.4/1.0 | 1.4/1.0 |
| Cook Temperature (Third Stage) | 80°C | 80°C | 80°C |
| Cook Time (Minutes) (Third Stage) | 90 | 90 | 90 |

**Table 6**

| Product Properties and Compositions for Examples 7-9 | | | |
|---|---|---|---|
| | Example 7 | Example 8 | Example 9 |
| MeO % | 35.4 | 36.1 | 34.9 |
| MeO DS | 2.20 | 2.25 | 2.17 |
| Viscosity (cP) | 461,000 | 26,000 | 25,000 |
| T_{gel} | 113°F (45°C) | 113°F (45°C) | 95°F (35°C) |
| G' (Pascals) | 6900 | 7990 | 7565 |
| Meltback time (minutes) | >50 | >50 | >50 |

G' - elastic modulus in Pascals in a 1.5 percent aqueous solution. Viscosity - viscosity in 2 percent aqueous solution in centipoise (cP). MeO % - percent methoxyl substitution based upon weight of methylcellulose. MeO DS - methoxyl degree of substitution.

**Table 7**

| PROPERTIES OF CONVENTIONAL PRIOR ART METHYLCELLULOSES | | | | | |
|---|---|---|---|---|---|
| **Process** | **% MeO¹** | **2% visc** | **G'²** | **Meltback³** | **Gel Temp (C°)⁴** |
| conventional | 29.5 - 30.5 | 15 | 213 | 3 - 6 | 56 |
| conventional | 29.5 - 30.5 | 400 | 578 | 7 - 10 | 57 |
| Conventional | 29.5 - 30.5 | 1,500 | 760 | 8 - 10 | 56 |
| Conventional | 29.5 - 30.5 | 4,000 | 1,000 | 8 - 11 | 57 |
| conventional | 29.5 - 30.5 | 22,000 | 1,500 | 10 - 13 | 52 |
| conventional | 29.5 - 30.5 | 40,000 | 1,800 | 16 - 21 | 56 |

| | | | | | |
|---|---|---|---|---|---|
| ¹ Percent methoxyl substitution ² G' = dynamic elastic modulus on Bohlin Rheometer at 1.5% aq. ³ Meltback time (minutes) of gel from 1.5% solution ⁴ Thermal gelation temperature of 1.5% solution | | | | | |

## Claims

1. A process for making methylcellulose having a level of methoxyl substitution of 21 to 42 weight percent comprising the steps of: a) reacting a cellulose pulp with a first amount of aqueous alkaline hydroxide and a first amount of a methylating agent at reaction conditions sufficient to methylate the cellulose pulp to a first level of methoxyl substitution which is 20 percent or more of the total level of methoxyl substitution; and b) reacting the methylcellulose of first level of methoxyl substitution with a second amount of aqueous alkaline hydroxide to alkalize it to a second level of alkalization and adding to the methyl cellulose of second level of alkalization continuously or incrementally a second amount of a methylating agent at reaction conditions sufficient to methylate it to a second level of methoxyl substitution which is 40 percent or more of the total level of methoxyl substitution, such that the process is a two-stage process, 20 to 80 percent of the total level of methoxyl substitution is carried out in the first stage and 80 to 20 percent of the total level of methoxyl substitution is carried out in the second stage.

2. The process of Claim 1, wherein the second amount of methylating agent is added at 65°C to 120°C for 15 minutes or more.

3. The process of Claim 1 or 2, wherein the methylation reaction is carried out at a temperature of 65°C to 120°C after addition of the second amount of methylating agent.

4. The process of Claim 1, wherein the process is a two-stage process, 40 to 60 percent of the total level of methoxyl substitution being carried out in the first stage and 60 to 40 percent of the total level of methoxyl substitution being carried out in the second stage.

5. The process of Claim 1, wherein contact with the cellulose pulp or the methylcellulose is effected in the first or the second stage with an amount of propylene oxide at reaction conditions sufficient to form a methylcellulose with hydroxypropyl substitution.

6. The process of Claim 1, wherein contact with the cellulose pulp or the methylcellulose is effected in the first or the second stage with an amount of butylene oxide at reaction conditions sufficient to form a methylcellulose with hydroxybutyl substitution.

7. The process of Claim 1, wherein contact with the cellulose pulp or the methylcellulose is effected in the first or the second stage with an amount of ethylene oxide at reaction conditions sufficient to form a methylcellulose with hydroxyethyl substitution.

8. The process of Claim 1, wherein contact with the cellulose pulp or the methylcellulose is effected in the first or the second stage with an amount of ethyl chloride at reaction conditions sufficient to form a methylcellulose with ethoxyl substitution.

9. A process for making methylcellulose having a level of methoxyl substitution of 21 to 42 weight percent comprising the steps of: a) reacting a cellulose pulp with a first amount of aqueous alkaline hydroxide and a first amount of a methylating agent at reaction conditions sufficient to methylate the cellulose pulp to a first level of methoxyl substitution which is 20 percent or more of the total level of methoxyl substitution; and b) reacting the methylcellulose of first level of methoxyl substitution with a second amount of aqueous alkaline hydroxide to alkalize it to a second level of alkalization and adding to the methyl cellulose of second level of alkalization continuously or incrementally a second amount of a methylating agent at reaction conditions sufficient to methylate it to a second level of methoxyl substitution which is 40 percent or more of the total level of methoxyl substitution, and
the process is a three-stage process, the process comprising the additional steps of:
contacting the methylcellulose of the second level of methoxyl substitution with a third amount of aqueous alkaline hydroxide to alkalize it to a third level of
contacting the methylcellulose of the third level of alkalization with a third amount of methylating agent..

10. The process of Claim 9, wherein 20 to 60 percent of the total level of methoxyl substitution is effected in each of the first and second stages and 5 to 30 percent in the third stage.

11. The process of any one of Claims 1 to 10, wherein the methylcellulose has a methoxyl substitution of 25 to 35 weight percent.

12. The process of any one of Claims I to 11, wherein the process further comprises c) dipping a pin into a solution of the methylcellulose of the total level of methoxyl substitution; d) allowing the solution to gel on the pins; e) drying the solution to form thin, dried layers of methylcellulose in the form of caps and bodies which are capable of being mated to form pharmaceutical capsules; and f) removing the caps and the bodies from the pins.

13. A methylcellulose made according to the process of any one of Claims 1 to 12.

14. A methylcellulose having a methoxyl substitution of 21 to 42 weight percent based upon the weight of the methylcellulose and a gel strength greater than 223 x (v ^{0.273}), wherein v represents the viscosity of the methylcellulose for a 2 percent aqueous solution at 20°C.

15. The methylcellulose of Claim 14 having a methoxyl substitution of 29 to 32 weight percent.

16. The methylcellulose of any one of Claims 13 to 15, wherein the gel strength is greater than 490 x (v ^{0.241}).

17. The methylcellulose of any one of Claims 13 to 16, wherein the methylcellulose has a viscosity of 1 to 600,000 centipoise in a two percent aqueous

18. The methylcellulose of any one of Claims 13 to 17, wherein the methylcellulose has a non-methoxyl substitution of 1 weight percent or less based upon the weight of the methylcellulose.

19. The methylcellulose of any one of Claims 13 to 18, wherein the methylcellulose is in the form of a pharmaceutical capsule or in the form of an excipient for a pharmaceutical tablet.

20. The methylcellulose of any one of Claims 13 to 18, wherein the methylcellulose is in the form of any of the following: a pharmaceutical encapsulant, capsule, tablet coating, or excipient; a drywall tape-joint compound; a mortar; a grout; a cement plaster; a spray plaster; a cement stucco; an adhesive; a paste; a wall/ceiling texturizers; a binder or processing aid for tape casting, extrusion forming, or injection molding; an agricultural spray adherent; a suspending/dispersing aid for pesticide, herbicide, or fertilizer powders; a shampoo; a lotion; a cleaner; or a ceramic.

21. A food composition comprising a methylcellulose according to any one of Claims 13 to 18.

22. The food composition according to Claim 21, wherein the food composition is selected from the group consisting of vegetable, meat, and soy patties; reformed seafood; reformed cheese sticks; cream soups; gravies and sauces; salad dressing; mayonnaise; onion rings; jams, jellies, and syrups; pie filling; formed potato products such as french fries and extruded fries; batters for fried foods, pancakes/waffles, and cakes; pet foods; beverages; frozen desserts; cultured dairy products such as ice cream, cottage cheese, yogurt, cheeses, and sour creams; cake icing and glazes; leavened and unleavened baked goods; and whipped topping.

23. The food composition of Claim 21 or 22, wherein the food composition

## Patentansprüche

1. Verfahren zur Herstellung von Methylcellulose, die einen Grad von Methoxylsubstitution von 21 bis 42 Gew.-% hat, umfassend die Stufen von:
a) Umsetzen einer Cellulosepulpe mit einer ersten Menge von wässrigem Alkalihydroxid und einer ersten Menge von einem Methylierungsmittel bei ausreichenden Reaktionsbedingungen zum Methylieren der Cellulosepulpe bis zu einem ersten Grad von Methoxylsubstitution, der 20% oder mehr des Gesamtgrades der Methoxylsubstitution beträgt; und
b) Umsetzen der Methylcellulose des ersten Grades von Methoxylsubstitution mit einer zweiten Menge von wässrigem Alkalihydroxid zu ihrem Alkalischmachen bis zu einem zweiten Grad von Alkalisierung und Zugabe zu der Methylcellulose des zweiten Grades der Alkalisierung einer zweiten Menge von einem Methylierungsmittel kontinuierlich oder in Teilmengen bei ausreichenden Reaktionsbedingungen zu ihrer Methylierung bis zu einem zweiten Grad von Methoxylsubstitution, welcher 40 % oder mehr des Gesamtgrades von Methoxylsubstitution ist, so dass das Verfahren ein Zweistufenverfahren ist, wobei 20 bis 80 % des Gesamtgrades der Methoxylsubstitution in der ersten Stufe durchgeführt werden und 80 bis 20 % des Gesamtgrades der Methoxylsubstitution in der zweiten Stufe durchgeführt werden.

2. Verfahren nach Anspruch 1, bei welchem die zweite Menge von Methylierungsmittel bei 65°C bis 120°C während 15 Minuten oder mehr zugegeben wird.

3. Verfahren nach Anspruch 1 oder 2, bei welchem die Methylierungsreaktion bei einer Temperatur von 65°C bis 120°C nach Zugabe der zweiten Menge von Methylierungsmittel durchgeführt wird.

4. Verfahren nach Anspruch 1, bei welchem das Verfahren ein Zweistufenverfahren ist, wobei 40 bis 60 % des Gesamtgrades der Methoxylsubstitution in der ersten Stufe durchgeführt werden und 60 bis 40 % des Gesamtgrades der Methoxylsubstitution in der zweiten Stufe durchgeführt werden.

5. Verfahren nach Anspruch 1, bei welchem der Kontakt mit der Cellulosepulpe oder der Methylcellulose in der ersten oder der zweiten Stufe mit einer Menge an Propylenoxid bei ausreichenden Reaktionsbedingungen zur Bildung einer Methylcellulose mit Hydroxypropylsubstitution durchgeführt wird.

6. Verfahren nach Anspruch 1, bei welchem der Kontakt mit der Cellulosepulpe oder der Methylcellulose in der ersten oder der zweiten Stufe mit einer Menge an Butylenoxid bei ausreichenden Reaktionsbedingungen zur Bildung einer Methylcellulose mit Hydroxybutylsubstitution durchgeführt wird.

7. Verfahren nach Anspruch 1, wobei der Kontakt mit der Cellulosepulpe oder der Methylcellulose in der ersten oder der zweiten Stufe mit einer Menge an Ethylenoxid bei ausreichenden Reaktionsbedingungen zur Bildung einer Methylcellulose mit Hydroxyethylsubstitution durchgeführt wird.

8. Verfahren nach Anspruch 1, bei welchem der Kontakt mit der Cellulosepulpe oder der Methylcellulose in der ersten oder der zweiten Stufe mit einer Menge an Ethylchlorid bei ausreichenden Reaktionsbedingungen zur Bildung einer Methylcellulose mit Ethoxylsubstitution durchgeführt wird.

9. Verfahren zur Herstellung von Methylcellulose, die einen Grad von Methoxylsubstitution von 21 bis 42 Gew.-% hat, umfassend die Stufen von:
a) Umsetzen einer Cellulosepulpe mit einer ersten Menge von wässrigem Alkalihydroxid und einer ersten Menge von einem Methylierungsmittel bei ausreichenden Reaktionsbedingungen zum Methylieren der Cellulosepulpe bis zu einem ersten Grad von Methoxylsubstitution, der 20 % oder mehr des Gesamtgrades der Methoxylsubstitution beträgt; und
b) Umsetzen der Methylcellulose des ersten Grades von Methoxylsubstitution mit einer zweiten Menge von wässrigem Alkalihydroxid zu ihrem Alkalischmachen bis zu einem zweiten Grad von Alkalisierung und Zugabe zu der Methylcellulose des zweiten Grades der Alkalisierung einer zweiten Menge von einem Methylierungsmittel kontinuierlich oder in Teilmengen bei ausreichenden Reaktionsbedingungen zu ihrer Methylierung bis zu einem zweiten Grad von Methoxylsubstitution, welcher 40 % oder mehr des Gesamtgrades von Methoxylsubstitution ist, und
bei welchem das Verfahren ein Dreistufenverfahren ist, wobei das Verfahren die zusätzlichen Stufen umfasst von:
Inkontaktbringen der Methylcellulose des zweiten Grades von Methoxylsubstitution mit einer dritten Menge von wässrigem Alkalihydroxid zu ihrem Alkalischmachen bis zu einem dritten Grad von Alkalisierung; und
Inkontaktbringen der Methylcellulose des dritten Grades von Alkalisierung mit einer dritten Menge von Methylierungsmittel.

10. Verfahren nach Anspruch 9, bei welchem 20 bis 60 % des Gesamtgrades von Methoxylsubstitution in jeder der ersten und zweiten Stufe sowie 5 bis 30 % in der dritten Stufe herbeigeführt werden.

11. Verfahren nach einem der Ansprüche 1 bis 10, bei welchem die Methylcellulose eine Methoxylsubstitution von 25 bis 35 Gew.-% hat.

12. Verfahren nach einem der Ansprüche 1 bis 11, bei welchem das Verfahren weiter umfasst:
c) Eintauchen eines Stiftes in eine Lösung der Methylcellulose von dem Gesamtgrad der Methoxylsubstitution;
d) Gelierenlassen der Lösung auf den Stiften;
e) Trocknen der Lösung zur Bildung dünner, getrockneter Schichten von Methylcellulose in Form von Kappen und Körpern, die zu pharmazeutischen Kapseln zusammengefügt werden können; und
f) Entfernen der Kappen und der Körper von den Stiften.

13. Methylcellulose, hergestellt nach dem Verfahren eines der Ansprüche 1 bis 12.

14. Methylcellulose, die eine Methoxylsubstitution von 21 bis 42 Gew.-%, bezogen auf das Gewicht der Methylcellulose, und eine Gelfestigkeit größer als 223 x (v^{0,273}) hat, wobei v die Viskosität der Methylcellulose für eine 2%ige wässrige Lösung bei 20°C darstellt.

15. Methylcellulose nach Anspruch 14, die eine Methoxylsubstitution von 29 bis 32 Gew.-% hat.

16. Methylcellulose nach einem der Ansprüche 13 bis 15, bei welcher die Gelfestigkeit größer als 490 x (v^{0,241}) ist.

17. Methylcellulose nach einem der Ansprüche 13 bis 16, wobei die Methylcellulose eine Viskosität von 1 bis 600.000 Centipoise in einer 2%igen wässrigen Lösung bei 20°C hat.

18. Methylcellulose nach einem der Ansprüche 13 bis 17, bei welcher die Methylcellulose eine Nicht-Methoxylsubstitution von 1 Gew.-% oder weniger, bezogen auf das Gewicht der Methylcellulose, hat.

19. Methylcellulose nach einem der Ansprüche 13 bis 18, bei welcher die Methylcellulose in Form einer pharmazeutischen Kapsel oder in Form eines Trägerstoffes für eine pharmazeutische Tablette vorliegt.

20. Methylcellulose nach einem der Ansprüche 13 bis 18, bei welcher die Methylcellulose in Form von irgendeinem der Folgenden vorliegt: einem pharmazeutischen Einkapselungsmittel, einer Kapsel, einem Tablettenüberzug, oder einem Trägerstoff; einer Trocken-Wandtapetenkleistermasse; einem Mörtel; einem Fugenmörtel; einem Zementputz; einem Sprühputz; einem Zementstuck; einem Klebstoff; einer Paste; einem Wand/Deckentextilmaterial; einem Bindemittel oder Verarbeitungshilfsstoff zum Foliengießen, zum Extrusionsformen oder Spritzgießen; einem landwirtschaftlichen Sprühkleber; einem Suspendier/Dispergierhilfsstoff für Pestizid-, Herbizid- oder Düngemittelpulver; einem Shampoo; einer Lotion; einem Reiniger; oder einer Keramik.

21. Lebensmittelzusammensetzung, umfassend eine Methylcellulose nach einem der Ansprüche 13 bis 18.

22. Lebensmittelzusammensetzung nach Anspruch 21, bei welcher die Lebensmittelzusammensetzung ausgewählt ist aus der Gruppe bestehend aus Gemüse-, Fleisch- und Sojapasteten; umgeformten Meeresfrüchten; umgeformten Käsestangen; Cremesuppen; Bratensäften und -soßen; Salatdressings; Mayonnaise; Zwiebelringen; Marmeladen, Gelees und Sirupen; Pastetenfüllung; geformten Kartoffelprodukten wie Kartoffelfritten und Kartoffelpuffern, Panaden für frittierte Lebensmittel; Pfannkuchen/Waffeln und Kuchen; Tierfutter; Getränken; gefrorenen Desserts; kultivierten Molkereiprodukten wie Eiscreme, Hüttenkäse, Joghurt, Käse und saure Sahne; Kuchenüberzügen und -glasuren; gebackenen Hefe- und Nichthefeprodukten; und aufgeschlagenen Produkten.

23. Lebensmittelzusammensetzung nach Anspruch 21 oder 22, bei welcher die Lebensmittelzusammensetzung von 0,01 bis 5 Gew.-% Methylcellulose, bezogen auf das Gesamtgewicht der Zusammensetzung, umfasst.

## Revendications

1. Procédé de préparation de méthylcellulose ayant un degré de méthoxylation de 21 à 42 % en poids, qui comprend les étapes suivantes :
a) faire réagir une pâte de cellulose avec une première quantité de solution aqueuse d'hydroxyde alcalin et une première quantité d'un agent de méthylation, dans des conditions de réaction permettant de méthyler la pâte de cellulose jusqu'à un premier degré de méthoxylation qui représente 20 % ou plus du degré total de méthoxylation, et
b) faire réagir la méthylcellulose ayant le premier degré de méthoxylation avec une seconde quantité de solution aqueuse d'hydroxyde alcalin pour l'alcaliniser jusqu'à un second degré d'alcalinisation, et ajouter à la méthylcellulose ayant le second degré d'alcalinisation, de manière continue ou par additions successives, une seconde quantité d'un agent de méthylation, dans des conditions de réaction permettant de la méthyler jusqu'à un second degré de méthoxylation qui représente 40 % ou plus du degré total de méthoxylation, de telle sorte que le procédé se déroule en deux étapes, 20 à 80 % du degré total de méthoxylation sont obtenus dans la première étape et 80 à 20 % du degré total de méthoxylation sont obtenus dans la seconde étape.

2. Procédé selon la revendication 1, dans lequel on ajoute la seconde quantité d'agent de méthylation à une température de 65 °C à 120 °C, en l'espace de 15 minutes ou plus.

3. Procédé selon la revendication 1 ou 2, dans lequel on effectue la réaction de méthylation à une température de 65°C à 120°C après l'addition de la seconde quantité d'agent de méthylation.

4. Procédé selon la revendication 1, qui est un procédé en deux étapes, 40 à 60 % du degré total de méthoxylation étant obtenus dans la première étape et 80 à 20 % du degré total de méthoxylation étant obtenus dans la seconde étape.

5. Procédé selon la revendication 1, dans lequel on réalise dans la première étape ou la seconde étape, la mise en contact de la pâte de cellulose ou de la méthylcellulose avec une certaine quantité d'oxyde de propylène, dans des conditions de réaction permettant de former une méthylcellulose portant des substituants hydroxypropyle.

6. Procédé selon la revendication 1, dans lequel on réalise dans la première étape ou la seconde étape, la mise en contact de la pâte de cellulose ou de la méthylcellulose avec une certaine quantité d'oxyde de butylène, dans des conditions de réaction permettant de former une méthylcellulose portant des substituants hydroxybutyle.

7. Procédé selon la revendication 1, dans lequel on réalise dans la première étape ou la seconde étape, la mise en contact de la pâte de cellulose ou de la méthylcellulose avec une certaine quantité d'oxyde d'éthylène, dans des conditions de réaction permettant de former une méthylcellulose portant des substituants hydroxyéthyle.

8. Procédé selon la revendication 1, dans lequel on réalise dans la première étape ou la seconde étape, la mise en contact de la pâte de cellulose ou de la méthylcellulose avec une certaine quantité de chlorure d'éthyle, dans des conditions de réaction permettant de former une méthylcellulose portant des substituants éthoxy.

9. Procédé de préparation de méthylcellulose ayant un degré de méthoxylation de 21 à 42 % en poids, qui comprend les étapes suivantes :
a) faire réagir une pâte de cellulose avec une première quantité de solution aqueuse d'hydroxyde alcalin et une première quantité d'un agent de méthylation, dans des conditions de réaction permettant de méthyler la pâte de cellulose jusqu'à un premier degré de méthoxylation qui représente 20 % ou plus du degré total de méthoxylation, et
b) faire réagir la méthylcellulose ayant le premier degré de méthoxylation avec une seconde quantité de solution aqueuse d'hydroxyde alcalin pour l'alcaliniser jusqu'à un second degré d'alcalinisation, et ajouter à la méthylcellulose ayant le second degré d'alcalinisation, de manière continue ou par additions successives, une seconde quantité d'un agent de méthylation, dans des conditions de réaction permettant de la méthyler jusqu'à un second degré de méthoxylation qui représente 40 % ou plus du degré total de méthoxylation,
lequel procédé est un procédé en trois étapes, le procédé comprenant les étapes supplémentaires suivantes :
mettre en contact la méthylcellulose ayant le second degré de méthoxylation avec une troisième quantité de solution aqueuse d'hydroxyde alcalin pour l'alcaliniser jusqu'à un troisième degré d'alcalinisation, et
mettre en contact la méthylcellulose ayant le troisième degré d'alcalinisation avec une troisième quantité d'agent de méthylation.

10. Procédé selon la revendication 9, dans lequel 20 à 60 % du degré total de méthoxylation sont obtenus dans chacune des première et seconde étapes, et 5 à 30 % sont obtenus dans la troisième étape.

11. Procédé selon l'une quelconque des revendications 1 à 10, dans lequel la méthylcellulose a un degré de méthoxylation de 25 à 35 % en poids.

12. Procédé selon l'une quelconque des revendications 1 à 11, qui comprend en outre c) l'immersion d'une broche dans une solution de la méthylcellulose ayant le degré total de méthoxylation, d) la gélification de la solution sur les broches, e) le séchage de la solution pour former des couches minces, séchées, de méthylcellulose sous la forme de capuchons et de corps qui peuvent être appariés pour former des capsules pharmaceutiques, et e) l'enlèvement des capuchons et des corps des broches.

13. Méthylcellulose préparée par le procédé selon l'une quelconque des revendications 1 à 12.

14. Méthylcellulose ayant un degré de méthoxylation de 21 à 42 % en poids par rapport au poids de la méthylcellulose, et présentant une rigidité de gel supérieure à 223 x (v^{0,273}), v désignant la viscosité de la méthylcellulose en solution aqueuse à 2 %, à 20 °C.

15. Méthylcellulose selon la revendication 14, qui a un degré de méthoxylation de 29 à 32 % en poids.

16. Méthylcellulose selon l'une quelconque des revendications 13 à 15, qui présente une rigidité de gel supérieure à 490 x (v^{0,241}).

17. Méthylcellulose selon l'une quelconque des revendications 13 à 16, qui présente une viscosité de 1 à 600 000 centipoises en solution aqueuse à 2 %, à 20 °C.

18. Méthylcellulose selon l'une quelconque des revendications 13 à 17, qui présente un degré de substitution non-méthoxy inférieur ou égal à 1 % en poids par rapport au poids de la méthylcellulose.

19. Méthylcellulose selon l'une quelconque des revendications 13 à 18, qui est sous la forme d'une capsule pharmaceutique ou sous la forme d'un excipient pour un comprimé pharmaceutique.

20. Méthylcellulose selon l'une quelconque des revendications 13 à 18, qui est sous l'une quelconque des formes suivantes : un encapsulant pharmaceutique, une capsule, un revêtement de comprimé, ou un excipient ; un produit ruban-joint pour paroi sèche ; un mortier ; un mortier liquide ; un plâtre-ciment ; un plâtre pour pulvérisation ; un stuc-ciment ; un adhésif ; une pâte ; un texturant pour murs/plafonds ; un liant ou auxiliaire de transformation pour la coulée de ruban, le formage par extrusion ou le moulage par injection ; un produit adhérant pour une composition pulvérisable pour l'agriculture ; un agent de mise en suspension ou de dispersion pour pesticides, herbicides ou fertilisants en poudre ; un shampooing ; une lotion ; un agent de nettoyage ; ou une céramique.

21. Composition alimentaire qui comprend une méthylcellulose selon l'une quelconque des revendications 13 à 18.

22. Composition alimentaire selon la revendication 21, qui est choisie parmi les bouchées aux légumes, à la viande et au soja, les aliments marins reconstitués, les barres de fromage reconstitué, les soupes à la crème, les jus et les sauces, les assaisonnements pour salade, la mayonnaise, les rondelles d'oignon, les confitures, les gelées et les sirops, la garniture de pâté en croûte, les produits façonnés à base de pomme de terre, tels que les pommes de terre frites françaises et les pommes de terre frites extrudées, les pâtes pour fritures, les crêpes, les gaufres et les gâteaux, les aliments pour animaux familiers, les boissons, les desserts congelés, les produits laitiers travaillés comme les glaces, le fromage de campagne, le yaourt, les fromages et les crèmes aigres, le glaçage pour gâteaux et les glaçures, les produits cuits avec ou sans levain, et les crèmes de dessus fouettées.

23. Composition alimentaire selon la revendication 21 ou 22, qui renferme 0,01 à 5 % en poids de méthylcellulose par rapport au poids total de la composition.
